# FASCICULE DE BREVET EUROPEEN

(11) **EP 0 577 718 B1**
(45) Date de publication et mention de la délivrance du brevet: **19.06.1996**
(21) Numéro de dépôt: 92909165.0
(22) Date de dépôt: 03.04.1992
(51) Int. Cl.: A61K 7/48

(54) **UTILISATION DE SPHINGOLIPIDES, DANS LA PREPARATION D'UNE COMPOSITION COSMETIQUE OU DERMOPHARMACEUTIQUE PROTEGEANT LA PEAU ET LES CHEVEUX CONTRE LES EFFETS NOCIFS DE LA POLLUTION ATMOSPHERIQUE**
Verwendung von Sphingolipid zur Bereitung einer kosmetiscen oder hautpharmazeutischen Zusammensetzung zum Schützen von Haut und Haaren gegen die durch Luftverschmutzung induzierte schädliche Ergebnisse
USE OF SPHINGOLIPIDS IN THE PREPARATION OF A COSMETIC OR DERMOPHARMACEUTICAL COMPOSITION PROTECTING THE SKIN AND HAIR AGAINST THE HARMFUL EFFECTS OF ATMOSPHERIC POLLUTION

(30) Priorité: 03.04.1991 FR 9104053
(43) Date de publication de la demande: 12.01.1994
(73) Titulaire: L'OREAL, F-75008 Paris (FR)
(72) Inventeur: MONTASTIER, Christiane, F-78600 Maisons-Laffitte (FR); GRIAT, Jacqueline, F-94480 Ablon (FR)
(74) Mandataire: Tonnellier, Jean-Claude
(86) Numéro de dépôt international: FR9200302
(87) Numéro de publication internationale: WO9217160

(56) Documents cités:
- WO-A-86/00015
- WO-A-88/00044
- FR-A- 2 652 002
- GB-A- 2 177 092
- PATENT ABSTRACTS OF JAPAN, vol. 11, no. 115 (C-415)(2562), 10 Avril 1987; & JP-A-61 260 008
- PATENT ABSTRACTS OF JAPAN, vol. 11, no. 349 (C-456)(2796), 14 Novembre 1987; & JP-A-62 120 308

## Description

L'invention a pour objet l'utilisation de sphingolipides ou analogues, éventuellement en association avec un agent chélatant, dans des compositions cosmétiques, hygiéniques ou dermopharmaceutiques protégeant la peau et les cheveux contre les effets nocifs de la pollution de l'atmosphère par les métaux lourds.

Les spécialistes considèrent actuellement qu'une des causes du vieillissement cellulaire est l'amoindrissement des capacités de défense contre les radicaux libres et les phénomènes d'oxydation qu'ils initient.

On sait que la toxicité des polluants gazeux de l'air, tels que le dioxyde de soufre, l'ozone et les oxydes d'azote, est liée notamment à leur activité d'initiateurs de radicaux libres qui provoquent chez les êtres vivants des dommages cellulaires ; voir par exemple H.MEHLHORN et coll., Free Rad. Res. Comms., Vol. 3, n° 1, p.193-197 (1987).

Les cellules vivantes possèdent divers moyens de défense contre les radicaux libres. En particulier, certains systèmes enzymatiques (tels que les superoxyde dismutases ou SOD et les glutathione réductases ou GR), détruisent les radicaux libres.

On sait par ailleurs que les métaux lourds (plomb, cadmium, mercure) sont des polluants atmosphériques dont les émissions ont notablement augmenté, notamment en milieu urbain ou industriel.

Outre certains effets toxiques qui leur sont propres, les métaux lourds ont la propriété de diminuer l'activité des moyens de défense cellulaire (SOD, GR) contre les radicaux libres ; voir par exemple R.S. Dwivedi, J.Toxicol.-Cut. & Ocular Toxicol. 6(3), 183,191 (1987).

Ainsi, les métaux lourds aggravent les effets toxiques des polluants atmosphériques gazeux en diminuant l'efficacité des moyens naturels de défense, et provoquent une accélération du phénomène de vieillissement cellulaire.

Cela est vrai en particulier pour la peau et le cuir chevelu, qui sont en contact direct et permanent avec le milieu extérieur.

Les effets nocifs des métaux lourds se traduisent notamment par un vieillissement accéléré de la peau, avec un teint manquant d'éclat, et formation précoce de rides ou ridules, et aussi par une diminution de la vigueur et un aspect terne des cheveux.

La présente invention a pour objet de remédier à ces effets, rectifs du point de vue esthétique et/ou sanitaire, qui résultent de la pollution de l'air par les métaux lourds.

On a en effet découvert qu'il est possible de protéger la peau et/ou les cheveux contre les effets nocifs de la pollution de l'atmosphère par les métaux lourds grâce à des compositions cosmétiques comprenant comme ingrédient actif un sphingolipide ou un analogue de sphingolipide. De tels ingrédients actifs avaient été utilisés auparavant, notamment comme agents émollients ; voir par exemple Patent Abstracts of Japan, Vol.11, n°115 (C-415) (2562), 10 Avril 1987, Patent Abstracts of Japan, Vol.11, n°349 (C-456) (2796), 14 Novembre 1987, WO-A-8600015 et FR-A-2652002.

L'invention a notamment pour objet l'utilisation d'au moins un sphingolipide ou analogue de sphingolipide comme ingrédient actif dans la préparation d'une composition dermopharmaceutique destinée à protéger la peau et/ou les cheveux contre les effets nocifs de la pollution de l'atmosphère par les métaux lourds.

Le sphingolipide utilisé selon l'invention peut être naturel ou synthétique. On sait que les sphingolipides naturels comprennent différentes classes de composés : les céramides, les sphingomyélines, les cérébrosides, les sulfatides et les gangliosides.

Parmi les sphingolipides naturels, on citera les céramides dont on sait qu'ils sont les constituants les plus importants des lipides de la couche cornée de l'épiderme ; les cérébrosides, les sphingomyélines, les sulfatides et les gangliosides sont présents notamment dans les membranes cellulaires des mammifères. Il existe des céramides et des cérébrosides commerciaux. Les sphingolipides naturels sont extraits notamment de plantes, de peaux animales (en particulier de porc), de cerveaux de bovins, d'oeufs, de cellules sanguines, etc... Ils peuvent être préparés notamment par extraction selon les procédés décrits dans les demandes de brevet japonais 86/260008 et 87/120308.

Les sphingolipides utilisés selon l'invention sont notamment ceux qui répondent à la formule (I)

R₁CO-NHCH(CH₂OR₂)-CHOH-R₃ (I)

dans laquelle R₁ représente un groupement alkyle, alcényle, hydroxyalkyle ou hydroxyalcényle ayant de 9 à 34 atomes de carbone, lesdits groupements hydroxyalkyle et hydroxyalcényle pouvant être estérifiés par un acide gras, éventuellement insaturé, ayant de 9 à 34 atomes de carbone, R₂ représente -H ou le reste d'un ose, d'une osamine, d'un oligosaccharide ou de la phosphorylcholine, ledit ose ou ledit oligosaccharide étant éventuellement substitué par un ou plusieurs groupements sulfates ou restes d'acide sialique, et R₃ représente un groupement aliphatique éventuellement insaturé ayant de 10 à 25 atomes de carbone, ledit groupement aliphatique pouvant être substitué en position alpha par un groupement hydroxy ou acyloxy dont l'acyle dérive d'un acide gras éventuellement insaturé ayant de 9 à 34 atomes de carbone. L'acide gras éventuellement insaturé est par exemple l'acide linoléique. Les oses ou motifs saccharidiques (généralement de 1 à 4) sont choisis par exemple parmi le glucose, le galactose, le sulfogalactose, la N-acétylgalactosamine. La préparation de certains sphingolipides synthétiques répondant à la formule (I) a été décrite dans la demande de brevet français de la demanderesse déposée le 21 Février 1991 sous le n° 91 02091, intitulée : "Céramides, leur procédé de préparation et leurs applications en cosmétique et en dermopharmacie". Ces composés sont préparés par acylation de la fonction amine d'une sphingosine ou d'une dihydrosphingosine. L'acylation est effectuée par exemple avec un chlorure d'acide, un anhydride mixte, un ester de paranitrophénol, un ester de N-hydroxysuccinimide, un ester de dicyclohexylcarbodiimide, un ester d'alkyle inférieur, ou encore un azolide tel qu'un imidazolide ou un pyrazolide. On peut citer, à titre de sphingolipides synthétiques,
la N-oléoyl dihydrosphingosine (formule I où R₁ = C₁₇H₃₃, R₂=H et R₃ = C₁₅H₃₁) et
la N-linoléoyl dihydrosphingosine (formule (I) où R₁ = C₁₇H₃₁, R₂ = H et R₃ = C₁₅H₃₁), ces composés pouvant
être préparés notamment comme décrit dans la demande de brevet français 91 02091 déposée le 20 Février 1991.

Les sphingolipides commerciaux d'origine naturelle sont le plus souvent des mélanges de différents types de sphingolipides, pouvant contenir en outre des phospholipides.

Dans la composition obtenue selon l'invention, le sphingolipide peut être associé à au moins un phospholipide.

Parmi les analogues de sphingolipides, on citera en particulier ceux qui sont décrits dans la demande de brevet internationale WO 86/00015, dans la demande de brevet EP 277 641 et dans les demandes de brevet FR 86 09125 et 89 12423.

Dans les compositions préparées selon l'invention, le sphingolipide ou analogue de sphingolipide est présent à une concentration de 0,05 à 2 % en poids, et en particulier de 0,1 à 0,5 % en poids.

L'invention a également pour objet un procédé de traitement cosmétique, caractérisé par le fait que, dans le but de remédier aux effets inesthétiques, sur la peau ou les cheveux, de la pollution de l'atmosphère par les métaux lourds, et notamment aux effets se traduisant par un vieillissement accéléré de la peau, une diminution de la vigueur et un aspect terne des cheveux, on applique sur la peau, sur le cuir chevelu ou sur les cheveux une composition cosmétique ou hygiénique contenant comme ingrédient actif au moins un sphingolipide ou un analogue de sphingolipide tel que défini précédemment.

Pour préparer une composition cosmétique, hygiénique ou dermopharmaceutique destinée à protéger la peau et/ou les cheveux contre les effets nocifs de la pollution de l'atmosphère par les métaux lourds, on incorpore au moins un sphingolipide ou un analogue de sphingolipide, éventuellement en association avec un agent chélatant, dans un véhicule approprié.

Pour préparer la composition de l'invention, on peut notamment dissoudre le sphingolipide ou analogue de sphingolipide dans un véhicule approprié, notamment une phase grasse, alcoolique ou hydroalcoolique. En outre, si désiré, la phase grasse est mélangée à une phase aqueuse ou dispersée dans celle-ci.

La composition obtenue est par exemple une émulsion, une émulsion gélifiée, une lotion hydroalcoolique ou oléoalcoolique, une dispersion vésiculaire, une composition biphase, un spray ou une mousse aérosol.

Ces compositions sont préparées selon les méthodes usuelles. Elles se présentent notamment sous la forme de lait, de crème ou de composition bi-phase pour les soins de la peau ou des cheveux, ou encore sous forme de shampooing. Lorsque les compostions obtenues selon l'invention se présentent sous la forme de lait ou de crème, il s'agit d'émulsions du type eau-dans-l'huile ou huile-dans-l'eau, ou encore de dispersions aqueuses de sphérules lipidiques constituées de couches moléculaires organisées renfermant une phase aqueuse encapsulée.

Lorsque les compositions sont des compositions bi-phases, elles sont constituées par une phase inférieure aqueuse et une phase supérieure huileuse comprenant le sphingolipide. Le rapport pondéral entre la phase inférieure et la phase supérieure est compris par exemple entre 30:70 et 60:40.

Lorsque la composition obtenue selon l'invention se présente sous forme d'une dispersion vésiculaire, les lipides constitutifs des vésicules peuvent être des lipides ioniques, non ioniques ou leurs mélanges.

Selon un mode de réalisation particulier de l'invention, on utilise le sphingolipide ou analogue de sphingolipide en association avec un agent chélatant, c'est-à-dire avec un agent capable de former avec les cations métalliques soit des liaisons de coordination, soit des liaisons ioniques. Parmi les cations métalliques, on peut citer le plomb, le mercure et le cadmium. Parmi les agents chélatants formant des liaisons de coordination avec les cations métalliques, on citera notamment les dérivés phosphoniques, les acides polyiminoacétique ou polyaminoacétique tels que le DTPA(acide diéthylènetriaminopentacétique) ; l'acide phytique ; les amides tels que le N'- 5- 4- 5-(acétylhydroxyamino)pentyl amino -1,4- dioxobutyl hydroxyamino pentyl -N-(5-aminopentyl)-N-hydroxybutane diamide ou déféroxamine, la polythiourée et les polyéthylène imines.

Parmi les dérivés phosphoniques, on citera en particulier : l'acide aminotri(méthylènephosphonique), l'acide 1-hydroxyéthylidène 1,1-diphosphonique, l'acide éthylènediamine tétra(méthylènephosphonique), l'acide hexaméthylènediamine tétra(méthylènephosphonique) et l'acide diéthylènetriamine penta(méthylènephosphonique), et leurs sels.

Parmi les agents chélatants formant des liaisons ioniques avec les cations métalliques, on peut citer les alginates, de préférence de sodium, et l'agarose.

Dans la composition obtenue selon l'invention, la teneur en agent chélatant est généralement inférieure à 1 % en poids.

La composition peut contenir par exemple de 0,05 à 1 % en poids, et en particulier de 0,1 à 0,3 % en poids de l'agent chélatant.

Les compositions obtenues selon l'invention sont appliquées sur la peau, le cuir chevelu ou les cheveux en quantités et selon les méthodes usuelles.

Les exemples suivants illustrent l'invention sans toutefois la limiter.

### EXEMPLE 1 : Crème protectrice pour la peau

On a préparé selon les méthodes usuelles une crème de composition pondérale suivante :

| | |
|---|---|
| Tri-stéarate de sorbitan | 0,9 |
| Mono-di-tri-palmito-stéarate de glycéryle vendu sous la dénomination commerciale de "Monoglycérylstéarate MGSB" par la Société NIKKOL | 3 |
| Myristate de myristyle | 5 |
| Palmitate d'éthyl-2 hexyle | 4 |
| Isoparaffine (6-8-moles d'isobutylène)hydrogénée vendue sous la dénomination commerciale "Polysynlane" par la Société NIPPON OIL FATS | 2,5 |
| Sphingolipide | 0,25 |
| Complexant phosphonique (Dequest 2046) | 0,1 |
| Diméthylpolysiloxane vendu sous la dénomination "Volatile Silicone 7158" par la Société UNION CARBIDE | 5 |
| Alcool cétylique (C₁₆ 90 %) | 2,5 |
| Stérarate de polyéthylèneglycol, polyoxyéthyléné à 40 moles d'oxyde d'éthylène | 2 |
| Conservateurs | qs |
| Parfum | qs |
| Eau déminéralisée stérilisée, qsp | 100 |

Le sphingolipide est celui vendu sous la dénomination GLYCOCER par WAITAKI.

Le complexant phosphonique Dequest 2046 est le sel de sodium de l'acide éthylènediamine tétra(méthylènephosphonique) vendu par MONSANTO.

De façon analogue, on a préparé des crèmes contenant, au lieu du Dequest 2046, l'un des chélatants suivants : DTPA et acide phytique.

### EXEMPLE 2 : Composition bi-phase

Une composition bi-phase est obtenue en additionnant dans un flacon 50 % de la phase huileuse (A) et 50 % de la phase aqueuse (B) contenant les ingrédients suivants (parties en poids) :

### A. Phase huileuse

| | |
|---|---|
| Sphingolipide (GLYCOCER) | 0,1 |
| Octyldodécanol | 10 |
| Diméthylpolysiloxane vendu sous la dénomination "Volatile silicone 7158" par la Société UNION CARBIDE | 60 |
| Myristate d'isopropyle | qsp 100 |

### B. Phase aqueuse

| | |
|---|---|
| Condensat d'oxyde d'éthylène et d'oxyde de propylène vendu sous la dénomination commerciale "Symperonic PE/F87" par la Société ICI | 0,5 |
| Dihydrogénophosphate de potassium | 0,1 |
| Hydrogénophosphate dipotassique anhydre | 0,3 |
| Chlorure de sodium anhydre | 0,9 |
| Propylène glycol | 5 |
| Dequest 2046 | 0,3 |
| Eau déminéralisée | qsp 100 |

### EXEMPLE 3 : Composition bi-phase

Elle est similaire à celle de l'exemple 2, en remplaçant le sphingolipide GLYCOCER par 0,1 g de N-oléoyldihydrosphingosine.

### EXEMPLE 4 : Etude du rôle protecteur vis-à-vis de la cytotoxicité induite par les métaux lourds

Ce test a été effectué sur des cultures de fibroblastes et sur des cultures de kératinocytes.

On détermine la cytotoxicité induite par des doses croissantes de métaux lourds (cadmium, plomb, mercure).

On mesure la viabilité cellulaire par on colorant vital, le bromure de 3-(4,5-diméthylthiazol-2-yl)2,5-diphényl tétrazolium, ou MTT, composé jaune à l'état oxydé, qui est réduit par les différentes enzymes mitochondriales des cellules vivantes en formazan bleu-violet insoluble ; voir F.Denizot et R.Lang, J.Immunol. Methods, 89, 271-277 (1986).

Après addition du toxique étudié, sous la forme de sel soluble (chlorure, sulfate ou acétate), à la culture cellulaire, on évalue la cytotoxicité au bout de 24 heures.

Pour cela, on ajoute le MTT (à raison de 5 mg/ml) puis on provoque la lyse des cellules par addition d'isopropanol contenant 0,04 N HCl qui solubilise le formazan produit. On évalue la quantité de formazan (qui est formée uniquement par les cellules vivantes) par spectrométrie à 570 et 630 nm.

Par ailleurs, pour estimer les atteintes membranaires des cellules, on effectue un dosage de la lactodéshydrogénase (LDH) intra- et extracellulaire. Pour doser la LDH extracellulaire, on prélève une partie du milieu de culture et on utilise le kit BOEHRINGER (référence 124907).

Pour doser la LDH intracellulaire, on élimine le milieu de culture puis on provoque la lyse des cellules par du TRITON 100 à 0,2 %. Le lysat obtenu est utilisé pour doser la LDH intracellulaire à l'aide du kit BOEHRINGER.

L'agent chélatant étudié était le DEQUEST 2046 vendu par MONSANTO.

Le sphingolipide était le GLYCOCER vendu par WAITAKI.

### Résultats

### a) Sur les fibroblastes :

### - Test MTT :

Le sphingolipide dénommé GLYCOCER, utilisé dans les exemples 1 et 2, a un effet protecteur vis-à-vis du cadmium et du mercure à la dose de 5.10⁻⁴ (Poids/volume : poids du produit commercial en g/volume (en ml) du milieu de culture (M.E.M).

La N-oléoyldihydrosphingosine a un effet protecteur vis-à-vis du cadmium et du plomb à la dose de 1.10⁻⁶ (poids/volume : poids du produit synthétique en g/volume (en ml) du milieu de culture M.E.M.)

L'agent chélatant a un effet protecteur vis-à-vis du cadmium à la dose de 5.10⁻⁵ (vol/vol (volume du produit commercial en ml/volume du milieu de culture M.E.M. en ml)).

### - Test LDH :

Le sphingolipide GLYCOCER a un effet protecteur vis-à-vis du cadmium, du plomb et du mercure à la dose de 5.10⁻⁴ (Poids/volume).

L'agent chélatant a un effet protecteur vis-à-vis du cadmium et du plomb à la dose de 5.10⁻⁵ (vol/vol)

### b) Sur les kératinocytes :

### - Test MTT :

Le sphingolipide GLYCOCER a un effet protecteur vis-à-vis du cadmium et du mercure, à la dose indiquée précédemment.

Le chélatant a un effet protecteur vis-à-vis du cadmium à la dose indiquée précédémment.

### - Test LDH :

Aux doses indiquées précédemment, le sphingolipide GLYCOCER a un effet protecteur vis-à-vis des trois métaux lourds étudiés, et le chélatant a un effet protecteur vis-à-vis du cadmium et du plomb.

## Revendications

1. Utilisation d'au moins un sphingolipide ou d'un analogue de sphingolipide comme ingrédient actif dans la préparation d'une composition cosmétique, hygiénique ou dermopharmaceutique destinée à protéger la peau, le cuir chevelu et/ou les cheveux contre les effets nocifs de la pollution de l'atmosphère par les métaux lourds.

2. Utilisation selon la revendication 1, caractérisée par le fait que ledit sphingolipide est un sphingolipide naturel.

3. Utilisation selon la revendication précédente, caractérisée par le fait que ledit sphingolipide est choisi parmi les céramides, les cérébrosides, les sphingomyélines, les sulfatides et les gangliosides.

4. Utilisation selon l'une quelconque des revendications précédentes, caractérisée par le fait que ledit sphingolipide est un sphingolipide synthétique.

5. Utilisation selon l'une quelconque des revendications précédentes, caractérisée par le fait que ledit sphingolipide répond à la formule (I) :
R₁CO-NHCH(CH₂OR₂)-CHOH-R₃ (I)
dans laquelle R₁ représente un groupement alkyle, alcényle, hydroxyalkyle ou hydroxyalcényle ayant de 9 à 34 atomes de carbone, lesdits groupements hydroxyalkyle et hydroxyalcényle pouvant être estérifiés par un acide gras, éventuellement insaturé, ayant de 9 à 34 atomes de carbone, R₂ représente -H ou le reste d'un ose, d'une osamine, d'un oligosaccharide ou de la phophorylcholine, ledit ose ou ledit oligosaccharide étant éventuellement substitué par un ou plusieurs groupements sulfates ou restes d'acide sialique, et R₃ représente un groupement aliphatique éventuellement insaturé ayant de 10 à 25 atomes de carbone, ledit groupement aliphatique pouvant être substitué en position alpha par un groupement hydroxy ou acyloxy dont l'acyle dérive d'un acide gras éventuellement insaturé ayant de 9 à 34 atomes de carbone.

6. Utilisation selon l'une quelconque des revendications précédentes, caractérisée par le fait que ledit sphingolipide ou analogue de sphingolipide est employé en association avec au moins un phospholipide.

7. Utilisation selon l'une quelconque des revendications précédentes, caractérisée par le fait que ledit sphingolipide ou analogue de sphingolipide est présent dans la composition à une concentration de 0,05 à 2 % en poids, et en particulier de 0,1 à 0,5 % en poids.

8. Utilisation selon l'une quelconque des revendications précédentes, caractérisée par le fait que ledit sphingolipide ou analogue de sphingolipide est dissous dans une phase grasse, alcoolique, oléoalcoolique ou hydroalcoolique.

9. Utilisation selon l'une quelconque des revendications précédentes, caractérisée par le fait que ladite composition est une émulsion, une émulsion gélifiée, une lotion hydroalcoolique ou oléoalcoolique, une dispersion vésiculaire, une composition bi-phase, un spray ou une mousse aérosol.

10. Utilisation selon l'une quelconque des revendications précédentes, caractérisée par le fait que ledit sphingolipide ou analogue de sphingolipide est utilisé en combinaison avec un agent chélatant.

11. Utilisation selon la revendication précédente, caractérisée par le fait que ledit agent chélatant est choisi parmi les dérivés phosphoniques, le DPTA, l'acide phytique et la déféroxamine.

12. Utilisation selon la revendication précédente, caractérisée par le fait que ledit dérivé phosphonique est choisi parmi l'acide aminotri (méthylènephosphonique), l'acide 1-hydroxyéthylidène 1,1-diphosphonique, l'acide éthylènediamine tétra(méthylènephosphonique), l'acide hexaméthylènediamine tétra(méthylènephosphonique) et l'acide diéthylènetriamine penta(méthylènephosphonique), et leurs sels.

13. Utilisation selon l'une quelconque des revendications 10 à 12, caractérisée par le fait que ladite composition contient de 0,05 à 1 % en poids, et en particulier de 0,1 à 0,3 % en poids dudit agent chélatant.

14. Procédé de traitement cosmétique, caractérisé par le fait que, dans le but de remédier aux effets inesthétiques, sur la peau ou les cheveux, de la pollution de l'atmosphère par les métaux lourds, et notamment aux effets se traduisant par un vieillissement accéléré de la peau, une diminution de la vigueur et un aspect terne des cheveux, on applique sur la peau, sur le cuir chevelu ou sur les cheveux une composition cosmétique ou hygiénique contenant comme ingrédient actif au moins un sphingolipide ou un analogue de sphingolipide tel que défini dans l'une quelconque des revendications 1 à 5 et 7 à 9.

15. Procédé selon la revendication précédente, caractérisé par le fait que, dans ladite composition, ledit sphingolipide ou analogue de sphingolipide est employé en association avec au moins un phospholipide et/ou en combinaison avec un agent chélatant.

16. Procédé selon la revendication 15, caractérisé par le fait que ledit agent chélatant est tel que défini dans l'une quelconque des revendications 11 à 13.

## Claims

1. Use of at least one sphingolipid or of a sphingolipid analogue as active ingredient in the preparation of a cosmetic, hygienic or dermopharmaceutical composition intended for protecting the skin, the scalp and/or hair against the harmful effects of the pollution of the atmosphere by heavy metals.

2. Use according to Claim 1, characterized in that the said sphingolipid is a natural sphingolipid.

3. Use according to the preceding claim, characterized in that the said sphingolipid is chosen from ceramides, cerebrosides, sphingomyelins, sulphatides and gangliosides.

4. Use according to any one of the preceding claims, characterized in that the said sphingolipid is a synthetic sphingolipid.

5. Use according to any one of the preceding claims, characterized in that the said sphingolipid corresponds to the formula (I):
R₁CO-NHCH(CH₂OR₂)-CHOH-R₃ (I)
in which R₁ represents an alkyl, alkenyl, hydroxyalkyl or hydroxylalkenyl group having from 9 to 34 carbon atoms, it being possible for the said hydroxyalkyl and hydroxyalkenyl groups to be esterified by an optionally unsaturated fatty acid having from 9 to 34 carbon atoms, R₂ represents -H or the residue of a monosaccharide, an osamine, an oligosaccharide or phosphorylcholine, the said monosaccharide or the said oligosaccharide optionally being substituted by one or a number of sulphate groups or sialic acid residues, and R₃ represents an optionally unsaturated aliphatic group having from 10 to 25 carbon atoms, it being possible for the said aliphatic group to be substituted in the alpha position by a hydroxyl or acyloxy group in which the acyl derives from an optionally unsaturated fatty acid having from 9 to 34 carbon atoms.

6. Use according to any one of the preceding claims, characterized in that the said sphingolipid or sphingolipid analogue is employed in combination with at least one phospholipid.

7. Use according to any one of the preceding claims, characterized in that the said sphingolipid or sphingolipid analogue is present in the composition at a concentration from 0.05 to 2 % by weight and in particular from 0.1 to 0.5 % by weight.

8. Use according to any one of the preceding claims, characterized in that the said sphingolipid or sphingolipid analogue is dissolved in a fatty, alcoholic, oil/alcoholic or aqueous/alcoholic phase.

9. Use according to any one of the preceding claims, characterized in that the said composition is an emulsion, a gelled emulsion, an aqueous/alcoholic or oil/alcoholic lotion, a vesicular dispersion, a two-phase composition, a spray or an aerosol foam.

10. Use according to any one of the preceding claims, characterized in that the said sphingolipid or sphingolipid analogue is used in combination with a chelating agent.

11. Use according to the preceding claim, characterized in that the said chelating agent is chosen from phosphonic derivatives, DPTA, phytic acid and deferoxamine.

12. Use according to the preceding claim, characterized in that the said phosphonic derivative is chosen from aminotri(methylenephosphonic) acid, (1-hydroxyethylidene)-1,1-diphosphonic acid, ethylene-diaminetetra(methylenephosphonic) acid, hexamethylene-diaminetetra(methylenephosphonic) acid and diethylene-triaminepenta(methylenephosphonic) acid, and their salts.

13. Use according to any one of Claims 10 to 12, characterized in that the said composition contains from 0.05 to 1 % by weight and in particular from 0.1 to 0.3 % by weight of the said chelating agent.

14. Process for cosmetic treatment, characterized in that, with the aim of overcoming the unaesthetic effects, on the skin or hair, of the pollution of the atmosphere by heavy metals, and in particular the effects which are reflected by an accelerated ageing of the skin and a reduction in the strength and a lacklustre appearance of the hair, a cosmetic or hygienic composition containing, as active ingredient, at least one sphingolipid or sphingolipid analogue as defined in any one of Claims 1 to 5 and 7 to 9 is applied to the skin, to the scalp or to the hair.

15. Process according to the preceding claim, characterized in that, in the said composition, the said sphingolipid or sphingolipid analogue is employed in combination with at least one phospholipid and/or in combination with a chelating agent.

16. Process according to Claim 15, characterized in that the said chelating agent is as defined in any one of Claims 11 to 13.

## Patentansprüche

1. Verwendung mindestens eines Sphingolipids oder einer analogen Verbindung des Sphingolipids als Wirkstoff bei der Herstellung einer kosmetischen, hygienischen oder dermatologisch pharmazeutischen Zubereitung, die zum Schutz der Haut oder Kopfhaut und/oder der Haare gegen schädliche Effekte der atmosphärischen Verschmutzung durch Schwermetalle bestimmt ist.

2. Verwendung nach Anspruch 1, dadurch gekennzeichnet, daß das Sphingolipid ein natürliches Sphingolipid darstellt.

3. Verwendung nach dem vorstehenden Anspruch, dadurch gekennzeichnet, daß das Sphingolipid aus Ceramiden, Cerebrosiden, Sphingomyelinen, Sulfatiden und Gangliosiden ausgewählt wird.

4. Verwendung nach einem der vorstehenden Ansprüche, dadurch gekennzeichnet, daß das Sphingolipid ein synthetisches Sphingolipid darstellt.

5. Verwendung nach einem der vorstehenden Ansprüche, dadurch gekennzeichnet, daß das Sphingolipid der Formel (I)
R₁CO-NHCH(CH₂OR₂)-CHOH-R₃ (I)
entspricht, worin
R₁ einen Alkyl-, Alkenyl-, Hydroxyalkyl- oder Hydroxyalkenylrest mit 9 bis 34 Kohlenstoffatomen bedeutet, wobei die Hydroxyalkyl- und Hydroxyalkenylreste durch eine gegebenenfalls ungesättigte Fettsäure mit 9 bis 34 Kohlenstoffatomen verestert sein können,
R₂ -H oder einen "ose"-Rest (Zucker- bzw. Saccharid), einen "osamin"-Rest (Aminozucker), ein Oligosaccharid oder Phosphorylcholin darstellt, wobei der "ose"-Rest oder das Oligosaccharid gegebenenfalls durch einen oder mehrere Sulfat- oder Sialinsäurereste substituiert sind, und
R₃ einen gegebenenfalls ungesättigten aliphatischen Rest mit 10 bis 25 Kohlenstoffatomen darstellt, wobei der aliphatische Rest gegebenenfalls in der α-Stellung durch eine Hydroxy- oder Acyloxygruppe substituiert ist, und sich der Acylrest von einer gegebenenfalls ungesättigten Fettsäure mit 9 bis 34 Kohlenstoffatomen ableitet.

6. Verwendung nach einem der vorstehenden Ansprüche, dadurch gekennzeichnet, daß das Sphingolipid oder die analoge Verbindung des Sphingolipids in Kombination mit mindestens einem Phospholipid angewendet wird.

7. Verwendung nach einem der vorstehenden Ansprüche, dadurch gekennzeichnet, daß das Sphingolipid oder die analoge Verbindung eines Sphingolipids in der Zubereitung in einer Konzentration von 0,05 Gew.-% bis 2 Gew.-%, inbesondere von 0,1 Gew.-% bis 0,5 Gew.-%, vorhanden ist.

8. Verwendung nach einem der vorstehenden Ansprüche, dadurch gekennzeichnet, daß das Sphingolipid oder die analoge Verbindung eines Sphingolipids in einer Fett-, Alkohol- oder Ölalkoholphase oder in wäßrigem Alkohol aufgelöst wird.

9. Verwendung nach einem der vorstehenden Ansprüche, dadurch gekennzeichnet, daß die Zusammensetzung eine Emulsion, eine gelierte Emulsion, eine wäßrig-alkoholische oder ölig-alkoholische Lotion, eine vesikuläre Dispersion, eine zweiphasige Zusammensetzung, ein Spray oder ein Aerosolschaum ist.

10. Verwendung nach einem der vorstehenden Ansprüche, dadurch gekennzeichnet, daß das Sphingolipid oder die analoge Verbindung des Sphingolipids in Kombination mit einem Komplexiermitel verwendet wird.

11. Verwendung nach dem vorstehenden Anspruch, dadurch gekennzeichnet, daß das Komplexiermittel aus Phosphonsäurederivaten, DPTA, Phytinsäure und Deferoxamin besteht.

12. Verwendung nach dem vorstehenden Anspruch, dadurch gekennzeichnet, daß das Phosphonsäurederivat aus der Aminotrimethylenphosphonsäure, 1-Hydroxyethyliden-1,1-diphosphonsäure, Ethylendiamintetramethylenphosphonsäure, Hexamethylendiamintetramethylenphosphonsäure, Diethylentriaminpentamethylenphosphonsäure und deren Salzen ausgewählt wird.

13. Verwendung nach einem der Ansprüche 10 bis 12, dadurch gekennzeichnet, daß die Zubereitung 0,5 Gew.-% bis 1 Gew.-%, insbesondere 0,1 Gew.-% bis 0,3 Gew.-%, an Komplexiermittel enthält.

14. Verfahren zur kosmetischen Behandlung, dadurch gekennzeichnet, daß zum Erzielen der Beseitigung unästhetischer Effekte auf der Haut oder den Haaren infolge der atmosphärischen Verschmutzung durch Metallsalze, insbesondere infolge von Effekten, die zu einer beschleunigten Hautalterung, einer verminderten Straffheit und einem matten Aussehen der Haare führen, auf der Haut, der Kopfhaut oder auf den Haaren eine kosmetische oder hygienische Zubereitung mit einem Gehalt mindestens an einem Sphingolipid oder einer analogen Verbindung des Sphingolipids als Wirkstoff gemäß Definition nach einem der Ansprüche 1 bis 5 und 7 bis 9 aufgebracht wird.

15. Verfahren nach dem vorstehenden Anspruch, dadurch gekennzeichnet, daß das Sphingolipid oder die analoge Verbindung eines Sphingolipids in der Zubereitung in Kombination mit mindestens einem Phospholipid und/oder in Kombination mit einem Komplexiermittel angewendet wird.

16. Verfahren nach Anspruch 15, dadurch gekennzeichnet, daß das Komplexiermittel die in einem der Ansprüche 11 bis 13 angegebene Bedeutung aufweist.
